# EUROPÄISCHE PATENTSCHRIFT

(11) **EP 4 062 848 B1**
(45) Veröffentlichungstag und Bekanntmachung des Hinweises auf die Patenterteilung: **22.10.2025**
(21) Anmeldenummer: 21164843.1
(22) Anmeldetag: 25.03.2021
(51) Int. Cl.: A61B 18/14, A61B 90/00, A61B 17/00

(54) **MEDIZINISCHES INSTRUMENT UND VERFAHREN ZUR HERSTELLUNG SOLCHER INSTRUMENTE**
MEDICAL INSTRUMENT AND METHOD FOR MANUFACTURING SUCH INSTRUMENTS
INSTRUMENT MÉDICAL ET PROCÉDÉ DESTINÉS À LA FABRICATION DE TELS INSTRUMENTS

(43) Veröffentlichungstag der Anmeldung: 28.09.2022
(73) Patentinhaber: Erbe Elektromedizin GmbH, 72072 Tübingen (DE)
(72) Erfinder: KAUPP, Stefan, 72072 Tübingen (DE); BOB, Felix, 72108 Rottenburg (DE)
(74) Vertreter: Rüger Abel Patentanwälte PartGmbB

(56) Entgegenhaltungen:
- EP-A1- 2 581 059
- WO-A2-2019/232375
- US-A1- 2011 177 474
- US-A1- 2013 218 150
- US-A1- 2014 316 401
- US-A1- 2017 049 506
- US-B2- 9 498 279

## Beschreibung

Die Erfindung betrifft ein medizinisches Instrument, sowie ein Verfahren zur Bereitstellung erfindungsgemäßer Instrumente.

Medizinische, am Patienten einzusetzende Instrumente bestehen häufig aus einer Vielzahl von Komponenten, die mehr oder weniger fest miteinander verbunden sind. Beispielsweise offenbart die WO 00/27294 A1 ein medizinisches Instrument zum Schneiden von Geweben in menschlichen oder tierischen Körper, das nach Art einer Schere ausgebildet ist. Die Schere weist Scherenarme auf, die aus Metall bestehen und mit einer elektrischen Isolierung versehen sind.

Aus der DE 10 2018 004 244 A1 ist ein autoklavierbarer Handgriff bekannt, der ein Gehäuse aufweist, in dem ein Schaltelement und weitere Elemente angeordnet sind. Das Schaltelement ist in dem Handgriffgehäuse über ein umlaufendes Dichtungselement abgedichtet. Um eine vollständige maschinelle Reinigung zu ermöglichen, ist das Gehäuse dieses Handgriffs frei von Fügestellen ausgebildet, wobei federelastisch in das Handgriffgehäuse eindrückbare Schaltelemente vorgesehen sind. Die umlaufende Dichtung ist aus einem autoklavierbaren Material ausgebildet und überbrückt den Spalt zwischen dem beweglichen Schaltelement und dem starren Handgriffgehäuse abdichtend. Die in dem Handgriff ausgebildeten Hohlräume sind somit von außen weitgehend unzugänglich.

Aus der DE 10 2004 041 871 B4 ist eine autoklavierbare Fernbedienung mit einem zweiteiligen Gehäuse bekannt, in dem eine mit Silikon und Vergussmasse ummantelte Platine angeordnet ist. Zur Herstellung der Fernbedienung wird zunächst die mit Bauelementen versehene Platine hermetisch in eine Vergussmasse eingegossen, wobei mechanische Elemente der Fernbedienung etwas aus der Vergussmasse herausstehen. Danach wird ein Oberteil mit der Vergussmasse verklebt, wobei mindestens ein Taster von einem definierten Lufteinschluss umgeben wird.

Aus der DE 694 18 799 T2 ist ein sterilisierbares zahnärztliches Handstück mit elektrischer Spule bekannt, das darauf eingerichtet ist, in zusammengebautem Zustand durch ein beliebiges aktuelles Sterilisationsverfahren sterilisiert zu werden. Das Handstück ist dabei so aufgebaut, dass ein Gehäuseelement oder Mantel sowie eine Spuleneinheit in zusammengebautem Zustand am Vorderende der Spuleneinheit lose zusammenpassen, damit Sterilisationsfluide, wie Dampf, Chemikalien oder Wärme für schnellere und gründlichere Sterilisation des gesamten Handstücks in dessen Inneres eindringen können. Dazu sollen an dem Handstück ein oder mehrere Öffnungen oder Spalte vorgesehen sein, die sich in das Innere des Handstücks erstrecken.

Aus der EP 1 769 764 A2 sowie der US 9 498 279 A2 ist jeweils ein elektrochirurgisches Instrument zum Schneiden von Gewebe bekannt, das zwei Scherenarme aufweist. Die Scherenarme sind jeweils als Hohlräume umschließende Gehäuse mit darin angeordneten Metallteilen ausgebildet. Die Gehäuse sind aus Gehäuseschalen zusammengesetzt, die miteinander verklebt sein können.

Die EP 2 630 982 A1 beschreibt einen Fluidstecker, über den ein Instrument mit einem Fluid zu versorgen ist. Der Stecker ist als Gehäuse ausgebildet, durch das sich einzelne Leitungen erstrecken. Der Innenraum des Steckers ist mit einer Vergussmasse gefüllt.

An elektrochirurgische Instrumente werden zahlreiche Forderungen gestellt. Neben einer zuverlässigen vielmaligen Sterilisierbarkeit kommt es häufig auch auf geometrische Präzision des Instruments und der an ihm vorhanden Funktionseinheiten und auf mechanische Belastbarkeit an. Beispielsweise müssen an dem Instrument vorhandene Elektroden in Bezug aufeinander oder in Bezug auf andere Elemente, wie beispielsweise Scharniere, präzise positioniert sein. Auch kommt es häufig auf eine gleichmäßige überall ausreichend dicke Isolierung an.

In WO 2019/232375 A2 wird ein chirurgisches System mit einem chirurgischen Handinstrument offenbart, das einen Sender für sichtbares Licht aufweist. Das chirurgische Handstück enthält ein Vergussmaterial, das zwischen dem Sender für das sichtbare Licht und dem ersten Ende des Gehäuses angeordnet ist. US 2014/316 401 A1 beschreibt ferner eine elektrochirurgische Vorrichtung, die einen länglichen Schaft mit einem distalen und einem proximalen Ende, einen elektrochirurgischen Endeffektor, eine elektrisch isolierende Hülle und ein elektrisch isolierendes viskoses Material aufweist. Zudem beschreibt US 2011/177 474 A1 ein magnetostriktives, sterilisierbares Handinstrument mit einem leitenden Draht, einem Gehäuse und einer isolierenden Überformungsschicht, die über dem leitenden Draht aufgebracht ist, um die Spule im Wesentlichen vollständig abzudichten, während der erste elektrische Kontakt und der zweite elektrische Kontakt frei bleiben, um mit einem Satz von externen elektrischen Kontakten in Eingriff zu kommen. Zudem beschreibt EP 2 581 059 A1 ein chirurgisches Instrument mit einem langen Schaft, in welchem ein Dichtelement angeordnet ist, das durch Urformen am Einbauort erzeugt worden ist. In US 2013/0218150 A1 wird ferner ein medizinisches Instrument mit einem Instrumentenstecker beschrieben, dessen Fluidstecker an einer, vorzugsweise ebenen, Stirnseite angeordnet sind.

Davon ausgehend ist es Aufgabe der Erfindung, ein verbessertes elektrochirurgisches Instrument und ein Verfahren zur Herstellung derselben anzugeben.

Diese Aufgabe wird mit dem Instrument nach Anspruch 1 und dem Verfahren nach Anspruch 12 gelöst:
Das erfindungsgemäße Instrument weist mindestens ein Griffstück auf, das aus einem Gehäuse mit mehreren Gehäuseschalen besteht. Die Gehäuseschalen umschließen einen Innenraum, in dem (mindestens) ein Innenteil, z.B. ein Metall-Inlay, angeordnet ist. Zwischen dem Innenteil und dem Gehäuse ist ein Zwischenraum ausgebildet, der frei von Lufteinschlüssen mit einer Vergussmasse gefüllt ist. Zwischen den Gehäuseschalen sind Fugen ausgebildet und die Fugen sind erfindungsgemäß vollständig mit der Vergussmasse gefüllt. Außerdem sind die Gehäuseschalen an den Stirnseiten profiliert.

Mit diesem Konzept lässt sich eine einfache und zugleich präzise Fertigung elektrochirurgischer Instrumente darstellen. Innenteile, beispielsweise stabilitätsgebende Metall-Inlays, Inlays aus Faserverbundmaterial, insbesondere faserverstärktem Kunststoff oder dergleichen, können in die Gehäuseschalen eingelegt und dann umgossen werden, wobei vorzugsweise leicht fließende und spaltfüllende Vergussmasse verwendet wird. Die Vergussmasse kann somit drucklos eingebracht werden, ohne dass die Gefahr der Dislozierung verwendeter Innenteile besteht. Anders als beim Einlegen von Metallteilen, Kabeln oder sonstigen Funktionselementen in eine Spritzgussform und Umspritzen der Teile mit zähplastischem Kunststoff, besteht bei dem erfindungsgemäßen Instrument, wie auch bei dem erfindungsgemäßen Verfahren, nicht die Gefahr der Ausschussproduktion infolge der Verlagerung oder Verdrängung von Innenteilen (Metall-Inlays, Kabeln, Schaltern und dergleichen) durch den beim Spritzgießen mit hohem Druck (bis zu 300 Bar) injizierten Kunststoff. Die verwendeten Innenteile können bedarfsweise an Stellen gehalten werden, die später als Funktionsteile freiliegend positioniert und gehalten werden. Auf diese Weise können beispielsweise Branchen von Scheren oder Koagulationszangen hergestellt werden, bei denen sich ein längliches Metallteil als Innenteil durch von distalen Ende aus über einen Scharnierbereich hinweg bis zum proximalen Ende hin erstreckt. Das Metallteil wird zunächst in das aus mehreren Gehäuseschalen zusammenzusetzende Gehäuse eingesetzt und dort beispielsweise am Scharnier oder an den später als Elektroden dienenden freiliegenden Bereichen positioniert, wonach die Vergussmasse in das Gehäuse eingefüllt und darin ausgehärtet wird.

Durch die komplette Ausfüllung des Innenraums mit Vergussmasse wird das Eindringen von Keimen, Schmutz oder anderen Verunreinigungen in dem Innenraum des Gehäuses verhindert. Spalte oder Hohlräume sind komplett ausgefüllt. Dies erleichtert die Sterilisation erheblich. Das Einbringen der noch flüssigen Vergussmasse in den Innenraum kann vor erfolgen, indem schalenförmige Gehäuseteile zunächst mit flüssiger Vergussmasse gefüllt, dann die Inlays eingelegt und danach die Gehäuseteile zusammengefügt werde. Vorzugsweise wird die Vergussmasse aber erst nach dem Zusammensetzen des Instruments in dessen Innenraum gefüllt. Dazu können in einer oder mehreren der Gehäuseschalen auch Einfüllöffnungen vorgesehen sein, durch die die Vergussmasse in den Innenraum eingebracht wird. Die Vergussmasse ist vorzugsweise eine bei der Aushärtung nicht schwindende, die Gehäuseschalen und die Innenteile klebend verbindende Masse, z.B**.** ein Gießharz.

Vorzugsweise bestehen die Gehäuseschalen aus einem Kunststoff, der steifer ist als die Vergussmasse. Die Vergussmasse kann aufgrund ihrer Nachgiebigkeit unterschiedliche thermische Ausdehnungskoeffizienten zwischen den Gehäuseschalen und den aus Metall bestehenden Innenteilen ausgleichen. Zwischen den Gehäuseschalen und dem Innenteil bilden sich keine Risse oder Spalten aufgrund thermischer Spannungen beim Autoklavieren, in die Keime eindringen könnten. Damit ist das Instrument auch vielmals sicher sterilisierbar.

Vorzugsweise sind die Gehäuseschalen miteinander an ihren Kanten, an denen sie sich gegenseitig berühren, spaltfrei, stoffschlüssig verbunden. Es handelt sich dabei vorzugsweise um eine unlösbare Verbindung, d.h**.** eine Verbindung, die sich nicht zerstörungsfrei lösen lässt. Die Gehäuseschalen können dazu mit einem Klebstoff miteinander verklebt sein, wodurch die Klebefugen nach außen abgedichtet sind. Die Vergussmasse wird dazu genutzt, die Gehäuseschalen miteinander zu verkleben. Zusätzlich können die Gehäuseschalen an ihren Stoßfugen miteinander verschweißt sein, beispielsweise durch Laserschweißen, Ultraschallschweißen, Reibschweißen oder dergleichen.

Das erfindungsgemäße Konzept gestattet die Ausbildung der Gehäuseschalen jeweils mit einheitlicher Wandstärke, beispielsweise im Spritzgussverfahren. Die Gehäuseschalen können so in hoher Präzision hergestellt werden. Die unterschiedlichen Abstände zwischen dem Innenteil und der Außenoberfläche der Gehäuseschalen werden durch die Vergussmasse überbrückt. Es ist auch möglich, die Gehäuseschalen in additiver Fertigungstechnik herzustellen. Insbesondere können dadurch Instrumente mit gleichen Innenteilen, aber unterschiedlichen Gehäusen bedarfsgerecht bereitgestellt werden (z.B. Instrumente für unterschiedlich große Hände oder für Rechts- und Linkshänder).

Das erfindungsgemäße Konzept ergibt besonders steife Griffstücke, weil die Gehäuseschalen aufgrund ihrer Formsteifigkeit zur Gesamtsteifigkeit des Griffstücks beitragen. Die Gehäuseschalen können somit zur Übertragung beispielweise von Betätigungskräften genutzt werden. Vorzugsweise bildet die Vergussmasse nach dem Aushärten eine kraftübertragende Verbindung zwischen dem Gehäuse und dem Innenteil, das vorzugsweise aus Metall besteht.

Das Innenteil kann nach außen komplett elektrisch isoliert und ohne elektrische Funktion sein. Es ist auch möglich, eine an dem Instrument vorgesehen Elektrode als Teil des Innenteils auszubilden. Das erfindungsgemäße Gestaltungskonzept lässt beides zu.

Das erfindungsgemäße Instrument kann zwei nach vorstehend erläutertem Prinzip ausgebildete Griffstücke aufweisen, die aneinander schwenkbar gelagert sind. Sie können beispielsweise die Branchen einer Zange oder Schere sein.

Das erfindungsgemäße Konzept gestattet außerdem den Aufbau eines Baukastensystems, mit dem sich aus einer Auswahl verschiedener Gehäuseschalen eines entsprechenden Vorrats verschiedene Instrument zusammenstellen lassen. Z.B. unterscheiden sich diese verschiedenen Instrumente untereinander lediglich hinsichtlich eines oder weniger Gehäusemerkmale. Weil sie hergestellt werden, ohne dass dazu jeweils für das gesamte Gehäuse des Instruments eine gesonderte Spritzgussform bereitgestellt werden müsste, sinkt der Fertigungsaufwand. Z.B. kann diejenige Gehäuseschale, die das variierende Gehäusemerkmal aufweist, in additiver Fertigung bereitgestellt werden. Das Gehäuse ist dann eine Kombination aus wenigstens einer additiv gefertigten Gehäuseschale und wenigstens einer im Spritzgussverfahren hergestellten Gehäuseschale. Unter einem additiven Fertigungsverfahren werden insbesondere ohne Gießform arbeitende Verfahren, wie z.B. 3D-Druck, selektives Laserhärten, selektives Lasersintern oder dergleichen verstanden. Es ist aber auch möglich, alle Gehäuseschalen im Spritzgussverfahren oder alle Gehäuseschalen in additiver Fertigungsweise herzustellen. Selbst wenn die Gehäuseschalen des in der Serie variierenden Gehäusemerkmals nicht additiv ohne Gießform sondern mittels einer Spritzgussform hergestellt werden, ergibt sich ein Effizienzvorteil. Beispielsweise können auf jede dieser genannten Weisen Koagulationszangen mit unterschiedlichen Handgriffgrößen bereitgestellt werden.

Weitere Einzelheiten vorteilhafter Ausführungsformen der Erfindung ergeben sich aus Ansprüchen sowie aus der Zeichnung und der zugehörigen Beschreibung. Die Figuren der Zeichnung zeigen:
Figur 1 ein elektrochirurgisches Instrument, in schematisierter perspektivischer Darstellung,
Figur 2 das Instrument nach Figur 1, in ausschnittsweiser perspektivischer Explosionsdarstellung,
Figur 3 eine Branche des Instruments nach Figur 1, im Querschnitt,
Figur 4 eine vergrößerte ausschnittsweise Darstellung des Querschnitts des Instruments nach Figur 3,
Figur 5 eine abgewandelte Ausführungsform einer Stoßfuge zwischen zwei Gehäuseschalen des Instruments nach Figur 1,
Figur 6 eine Elektrode mit daran angeschlossenem Kabel für ein Instrument nach Figur 1,
Figur 7 eine Querschnittsdarstellung des distalen Endbereichs einer abgewandelten Ausführungsform des Instruments nach Figur 1 und
Figur 8 bis 10 Griffschalen für den Handgriffbereich eines Baukastensystems zur Bereitstellung verschiedener Instrumente gleichen Typs aber unterschiedlicher Handgriffgröße in jeweils schematischer Perspektivdarstellung.

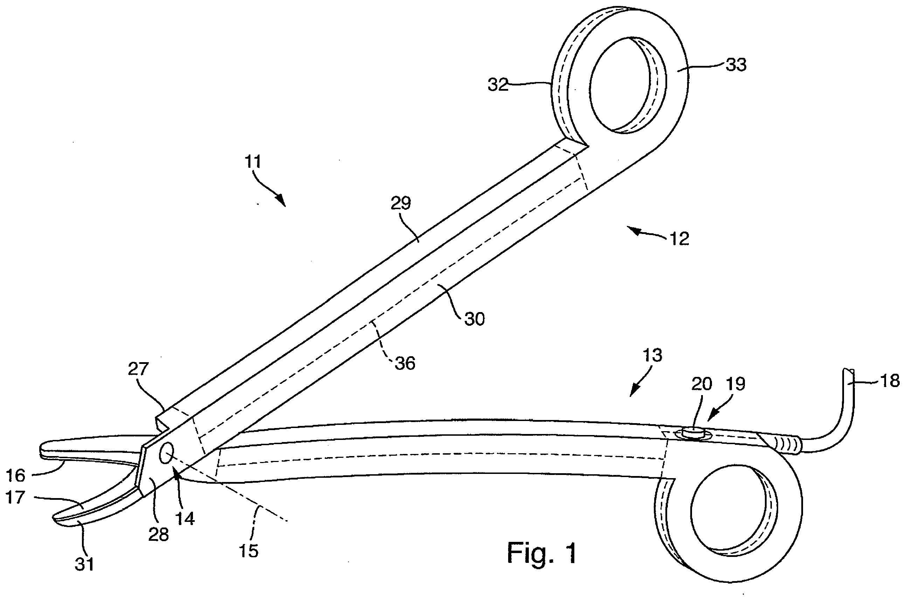

In Figur 1 ist ein Instrument 11 veranschaulicht, das hier beispielshalber als Kauterisierungszange für den offenchirurgischen Einsatz ausgebildet ist. Entsprechend dem nachfolgend erläuterten Aufbau und Bauprinzip des Instruments 11 können aber auch andere Instrumente, insbesondere für den offenchirurgischen Einsatz, ausgebildet sein, wie beispielsweise Gewebescheren oder kombinierte Koagulations- und Dissektionsinstrumente. Insbesondere handelt es sich dabei um Instrumente für den elektrochirurgischen Einsatz, bei dem mittels des Instruments Strom in Gewebe eingeleitet wird, um einen chirurgischen Effekt zu erzielen. Die erfindungsgemäß ausgebildeten Instrumente 11 sind vorzugsweise als wiederverwendbare, sterilisierbare Instrumente ausgebildet.

Das Instrument 11 weist mindestens ein oder, wie es in Figur 1 veranschaulicht ist, zwei (oder mehrere) Griffstücke 12, 13 auf, die bei dem Instrument 11 nach Figur 1 als zwei gelenkig aneinander gelagerten Branchen ausgebildet sind. Die Griffstücke 12, 13 sind dazu an einem Gelenk 14 schwenkbar aneinander gelagert. Das Gelenk 14 legt eine (einzige) Gelenkachse 15 fest, die quer zu den Griffstücken 12, 13 orientiert ist.

Das Instrument 11 weist mindestens eine, vorzugsweise mehrere, zum Beispiel zwei Elektroden 16, 17 auf, die an dem jeweiligen, sich von dem Gelenk 14 aus in distaler Richtung weg erstreckenden Teil dieses Griffstücks 12, 13 angeordnet sind. Die Elektroden 16, 17 sind gegeneinander isoliert und werden bedarfsweise über ein Kabel 18 mit Strom versorgt, das eine Verbindung zwischen den Elektroden 16, 17 und einem nicht veranschaulichten elektrochirurgischen Generator herstellt. Das Kabel 18 kann von einem proximalen Ende einer der beiden Griffstücke 12, 13 ausgehen. Bei anderen Ausführungsformen kann auch an jedem Griffstück 12, 13 eine entsprechende isolierte Leitung (Kabel) angeordnet sein.

Das Instrument 11 kann weitere elektrische oder elektronische Komponenten enthalten. Im einfachsten Fall ist beispielsweise an einem der Griffstücke 12, 13, vorzugsweise an demjenigen Griffstück 13, an dem das Kabel 18 angeordnet ist, ein elektrischer Schalter 19 vorgesehen, der dazu eingerichtet ist, den Stromfluss zu den Elektroden 16, 17 freizugeben oder zu sperren. Dieser optional vorgesehene Schalter 19 kann beispielsweise nach Art eines Tasters ausgebildet sein, der einen beweglichen Stößel 20 aufweist. Der Stößel 20 kann dem anderen Griffstück 12 zugewandt und so angeordnet sein, dass er betätigt wird, sobald das Griffstück 12 auf dem Stößel 20 aufsetzt und niedergedrückt wird.

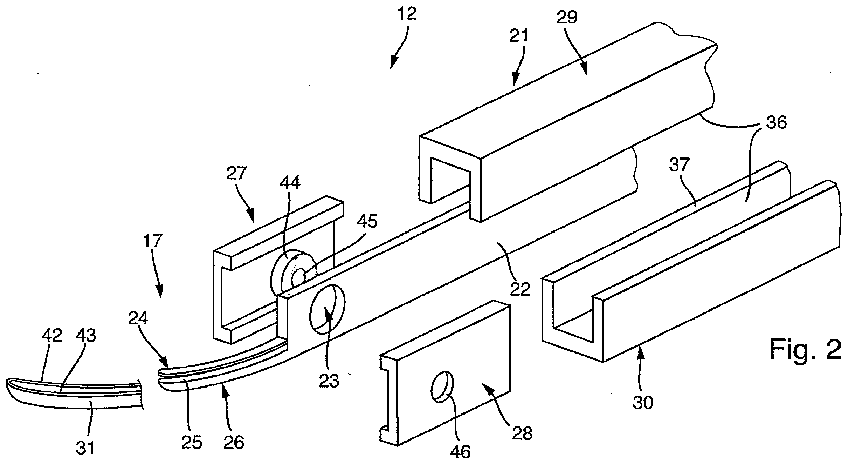

Die beiden Griffstücke 12, 13 sind nach dem in Figur 2 anhand des Griffstücks 12 erläuterten Prinzips aufgebaut. Das Griffstück 12 besteht aus einem mehrteiligen Gehäuse 21, das ein Innenteil 22 umgibt. Das Innenteil ist beispielsweise aus Metall ausgebildet und kann sich vom distalen Ende des Instruments 11 bis zum proximalen Ende desselben erstrecken. Das Innenteil 22 ist beispielsweise als zweiarmiger Hebel ausgebildet, der im Bereich des Gelenks 14 eine Öffnung 23 aufweist. Das distale Ende dieses Innenteils 22 kann einen Elektrodenabschnitt 24 aufweisen, der durch eine an beiden Flanken verlaufende Nut 25 von einem Trägerabschnitt 26 getrennt ist.

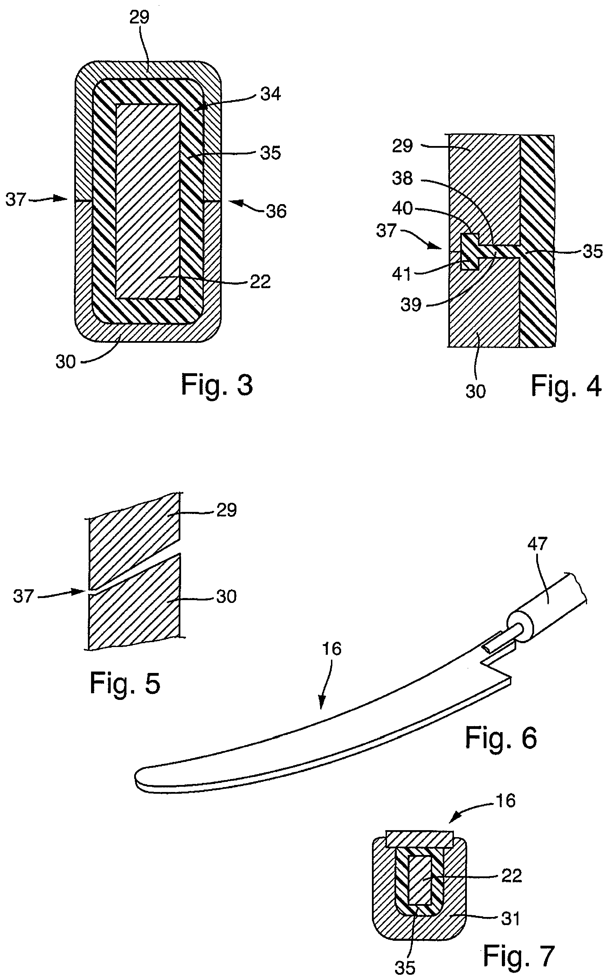

Das Gehäuse 21 besteht aus mehreren Gehäuseschalen 27 bis 31 sowie 32, 33 (Figur 1 und 2), die lückenlos zusammengefügt sind, um einen Innenraum 34 zu umschließen, in dem, wie in Figur 3 dargestellt, das Innenteil 22 angeordnet ist.

Die Gehäuseschalen 27 bis 33 sind vorzugsweise aus einem Kunststoff gefertigt, beispielsweise ein außen glatter und steifer Kunststoff, der für die üblichen Sterilisierungstemperaturen von beispielsweise 130°C oder bis zu 170°C temperaturbeständig ist. Die Gehäuseschalen 27 bis 33 können im Spritzgussverfahren hergestellt sein und weitgehend einheitliche Wandstärken aufweisen. Alternativ können einige oder alle der Gehäuseschalen 27 bis 33 in einem additiven Fertigungsprozess ohne Nutzung einer die Schalenform festlegenden Gießform hergestellt werden. Als Kunststoffefür die Gehäuseschalten 27 bis 33 eignen sich Polyetheretherketon (PEEK), Polyaryletherketone (PAEK), teilkristalline thermoplastische Konstruktionswerkstoffe z.B. auf Basis von Polyphthalamid (PPA), Polyarylamide (PARA) mit oder ohne Glasfaserverstärkung, Photopolymere auf Methacrylat-Basis oder andere Kunststoffe, die wenigstens bis 140°C temperaturbeständig sind.

Der von den Gehäuseschalen 27 bis 33 umgrenzte Innenraum 34 ist vorzugsweise mit einer Vergussmasse 35 gefüllt. Diese Vergussmasse 35 füllt den Innenraum 34 vorzugsweise vollständig und lückenlos aus. Dies gilt auch, wenn anstelle eines einzigen Innenteils 22 mehrere nebeneinander angeordnete oder aneinander angereihte Innenteile vorgesehen sind. Als Vergussmasse 35 eignet sich jeder temperaturbeständige aushärtbare Kunststoff, der mit dem Kunststoffmaterial der Gehäuseschalen 27 bis 33 eine stoffschlüssige Verbindung eingeht. Insbesondere kann die Vergussmasse 35 aus dem gleichen Kunststoff bestehen, wie die Gehäuseschalen 27 bis 33, insbesondere, wenn es sich um einen kalt aushärtbaren Kunststoff handelt. Die Vergussmasse 35 kann auch ein aushärtbarer Epoxidharzkleber oder eine Epoxidharz-Gießmasse, ein Cyankleber oder allgemein jeder Kleber mit einer Temperaturbeständigkeit von wenigstens 140 °C sein.

Das Gehäuse 21 ist nach außen vorzugsweise vollständig geschlossen. Die Gehäuseschalen 27 bis 33 stoßen dabei an in Figur 1 gestrichelt dargestellten und ansonsten auch aus Figur 3 bis 5 ersichtlichen Stoßfugen 36, 37 aneinander und sind vorzugsweise an den Stoßfugen 36, 37 miteinander unlösbar verbunden. Beispielsweise ist die verwendete Vergussmasse 35 in flüssigem, nicht ausgehärtetem Zustand geeignet, in die Stoßfugen einzudringen, wie es in Figur 4 beispielhaft anhand der Stoßfuge 37 dargestellt ist. Zur besseren und dichten Verbindung der Gehäuseschalen 27 bis 33 miteinander sind die Stirnseiten 38, 39 der Gehäuseschalen 29, 390 (sowie auch der weiteren Gehäuseschalen) profiliert. Beispielsweise können die Stirnseiten 38, 39 mit Längsnuten 40, 41 versehen sein, die sich beim Einfüllen der Vergussmasse 35 mit solcher füllen. Nach dem Aushärten der Vergussmasse 35 verklebt diese die Gehäuseschalen insgesamt und an der Stoßfuge 37 miteinander. Sie bildet in den Nuten 40, 41 eine Sperre gegen das Eindringen von Flüssigkeiten und Keimen in etwaige Spalte, die im Inneren des Instruments 11 noch vorhanden sein mögen.

Die Gestaltung der Stoßfuge 37 (sowie der weiteren Stoßfugen) kann variieren und von der genutzten Verbindungstechnik der Gehäuseschalen abhängen. Beispielsweise kann die Stoßfuge 37, wie in Figur 5 veranschaulicht, keilförmig und gegen die horizontale Fuge geneigt angeordnet sein, um ein leichtes Fügen einerseits und andererseits ein leichtes Füllen der Stoßfuge 37 mit Vergussmaterial zu ermöglichen.

Bei der insoweit beschriebenen Ausführungsform sind die Gehäuseschalen 27 bis 33 durch die Vergussmasse 35 miteinander verklebt. Es ist jedoch auch möglich, die Stoßfugen 36, 37 (und alle weiteren) zunächst mit einem Klebstoff gesondert zu verkleben und erst danach Vergussmasse in den Innenraum 34 einzufüllen. Weiter ist es möglich, die Stoßfugen 36, 37 anderweitig zu schließen und somit die Gehäuseschalen 27 bis 33 miteinander zu verbinden, beispielsweise durch Ultraschallschweißen, Laserschweißen, Reibschweißen oder dergleichen.

Die Gehäuseschalen 27 bis 33 sind verschiedenartig ausgebildet. Beispielsweise kann die Gehäuseschale 31 (Figur 2) dazu vorgesehen sein, den Trägerabschnitt 26 aufzunehmen, dabei aber den Elektrodenabschnitt 24 frei zu lassen. Die Gehäuseschale 31 kann somit als Schuh ausgebildet sein, der über den Trägerabschnitt 26 zu schieben ist. Die oberen Ränder 42, 43 der Gehäuseschale 31 können dabei eine nach innen vorspringende Leiste aufweisen, die in die Nut 25 passt. Weitere Gehäuseschalen 27, 28 können den Gelenkbereich des Instruments 11 einhüllen und bilden. Beispielsweise kann die Gehäuseschale 27 einen flachen U-förmigen Querschnitt sowie etwa mittig einen Fortsatz 44 aufweisen, der mit oder ohne Spiel in die Öffnung 23 passt. Der Fortsatz 44 kann eine Gelenköffnung 45 festlegen, die mit einer Öffnung 46 versehen ist, die mit der Gelenköffnung 45 fluchtet.

Die Gehäuseschalen 27, 28 legen ober- und unterhalb des Innenteils 22 angeordnet Stoßfugen fest. Proximal an die Gehäuseschalen 27, 28 anschließend sind die Gehäuseschalen 27, 30 vorgesehen, deren Stoßfugen 36, 37 horizontal sind. Am proximalen Ende sind die Gehäuseschalen 32, 33 angeordnet, die Öffnungen umschließen und somit als Fingergriffe ausgebildet sind.

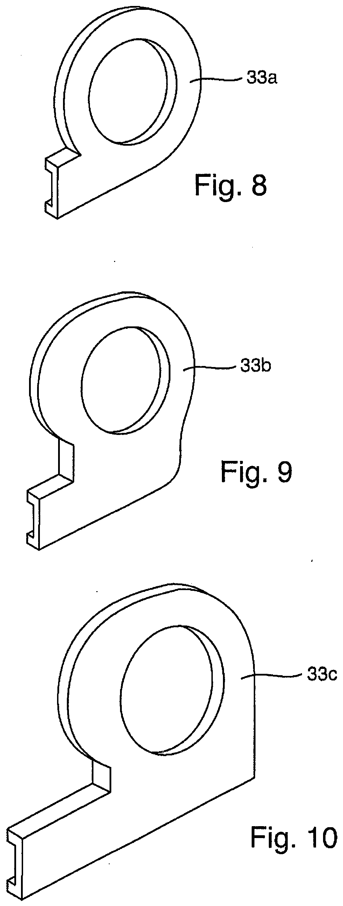

Die Gehäuseschalen 27 bis 33 können einen Gehäuseschalensatz bilden, der für ein und dieselbe Position des Gehäuses 21 unterschiedlich ausgebildete Gehäuseschalen umfasst. Dies ist am Beispiel der Gehäuseschale 33 in Figur 8 bis 10 veranschaulicht. Es sind dort verschiedene Gehäuseschalen 33a, 33b, 33c veranschaulicht, die gleichermaßen geeignet sind, an dem proximalen Ende des Gehäuses 21 und somit des Instruments 11 angeordnet zu werden. Die Gehäuseschalten 33a, 33b, 33c unterscheiden sich im Detail, zum Beispiel durch Größe und/oder Formgebung. Sie sind jedoch gleichermaßen geeignet, an die Gehäuseschalen 28, 30 anzuschließen. Auf diese Weise ist ein Gehäuseschalen-Bausatz geschaffen, mit dem sich auf einfache Weise Instrumente 11 mit unterschiedlichen Endformen oder unterschiedliche Fingergrößen herstellen lassen. Auch können auf diese Weise unterschiedliche Längen der Griffstücke 12, 13 oder Unterschiede hinsichtlich anderer Gehäusemerkmale realisiert werden.

Auch zu den anderen Gehäuseschalen 27 bis 31 können Alternativformen bereitgestellt werden, um unterschiedlich geformte Gehäuse zu realisieren.

An den vorgesehenen Instrumenten 11 sind Abwandlungen möglich. Während bei dem Instrument 11 nach Figur 2 die Elektrode 17 (und 16) durch das Innenteil 22, nämlich dessen Elektrodenabschnitt 24 gebildet werden ist, kann die Elektrode 16 (oder 17), wie die Figuren 6 und 7 nahelegen, auch als gesondertes, elektrisch von dem Innenteil 22 isoliertes Metallteil ausgebildet sein. An dieses kann eine isolierte Leitung 47 angeschlossen sein, die sich entlang des Innenteils 22 durch das Griffteil 13 bis zu dem Schalter 19 und/oder zu dem Kabel 13 erstreckt.

Das Instrument 11 wird wie folgt bereitgestellt.

Zunächst werden das Innenteil 22 und die Gehäuseschalen 27 bis 33 zusammengefügt. Sofern zu den Gehäuseschalen 27 bis 33 alternative Gehäuseschalen wie beispielsweise 33a bis 33c gemäß Figur 8 bis 10 gehören, werden aus diesem Vorrat die gewünschten Gehäuseschalen ausgewählt. Nach dem Zusammenfügen der Gehäuseschalen wird der entstandene Innenraum 34 vollständig mit Vergussmasse 35 aufgefüllt, die danach in dem Innenraum 34 zum Aushärten gebracht wird. Nach dem Aushärten der Vergussmasse 35 ist das Instrument 11 fertiggestellt und einsatzbereit.

Die Gehäuseschalen 27 bis 31 können aus gleichem Kunststoff oder auch aus unterschiedlichen Kunststoffen ausgebildet sein. Insbesondere kann beispielsweise für die Gehäuseschale 31 ein anderer Kunststoff gewählt werden als für die übrigen Gehäuseschalen. Entsprechendes gilt für die Gehäuseschalen 27, 28. Die Gehäuseschalen 27 bis 33 können jedoch auch aus gleichem Kunststoff ausgebildet sein. Vorzugsweise besehen sie aus einem temperaturbeständigen glatten Kunststoff. Die Vergussmasse 35 ist vorzugsweise in nicht ausgehärtetem Zustand dünnflüssig und nach ihrem Aushärten etwas elastisch. Vorzugsweise hat sie eine größere Elastizität als die Gehäuseschalen 27 bis 33. Sie verbindet das Innenteil 22 und die Gehäuseschalen 27 bis 33 dauerhaft und spaltfrei.

Aufgrund der Elastizität der Vergussmasse 35 sowie der Temperaturbeständigkeit derselben und des Gehäuses 21 kann das Instrument 11 thermisch wiederholt sterilisiert werden, ohne dass dadurch eine Degradation zu befürchten wäre.

Das vielmals sterilisierbares Instrument 11 weist Griffstücke 12, 13 auf, die als Gehäuse 21 ausgebildet sind. Dieses Gehäuse 21 besteht aus Gehäuseschalen 27 bis 33, die an Stoßfugen lückenlos aneinander gefügt sind und dessen Innenraum vollständig mit Vergussmasse 35 gefüllt ist. Das Instrument ist wenig verschmutzungsanfällig und leicht sterilisierbar.

### Bezugszeichen:

- 11: Instrument
- 12, 13: Griffstücke
- 14: Gelenk
- 15: Gelenkachse
- 16, 17: Elektroden
- 18: Kabel
- 19: Schalter
- 20: Stößel
- 21: Gehäuse
- 22: Innenteil
- 23: Öffnung
- 24: Elektrodenabschnitt
- 25: Nut
- 26: Trägerabschnitt
- 27 - 33: Gehäuseschalen
- 34: Innenraum
- 35: Vergussmasse
- 36, 37: Stoßfugen
- 38, 39: Stirnseiten
- 40, 41: Nuten
- 42, 43: Ränder der Gehäuseschale 31
- 44: Fortsatz
- 45: Gelenköffnung in dem Fortsatz 44
- 46: Öffnung passend zu der Gelenköffnung 45
- 47: Leitung

## Patentansprüche

1. Elektrochirurgisches Instrument (11), insbesondere thermisch sterilisierbares Instrument (11),
mit mindestens einem Griffstück (12, 13), das ein Gehäuse (21) aufweist, das aus mehreren Gehäuseschalen (27-33) zusammengesetzt ist und das ein Innenteil (22) umgibt, wobei zwischen dem Innenteil (22) und dem Gehäuse (21) ein Zwischenraum (34) ausgebildet ist, der mit einer Vergussmasse (35) gefüllt ist,
wobei zwischen den Gehäuseschalen (27-33) Fugen (36, 37) ausgebildet und die Gehäuseschalen (27-33) an den Stirnseiten (38, 39) profiliert sind, **dadurch gekennzeichnet, dass** die Fugen (36, 37) vollständig mit der Vergussmasse (35) gefüllt sind.

2. Instrument nach Anspruch 1, **dadurch gekennzeichnet,**
**dass** der Innenraum (34) vollständig frei von Lufteinschlüssen mit der Vergussmasse (35) gefüllt ist.

3. Instrument nach einem der vorstehenden Ansprüche,
**dadurch gekennzeichnet, dass** in dem Zwischenraum (34) wenigstens eine elektrische Komponente (19, 47) angeordnet ist.

4. Instrument nach einem der vorstehenden Ansprüche,
**dadurch gekennzeichnet, dass** die Gehäuseschalen (27-33) aus einem Kunststoff bestehen, der steifer ist als die Vergussmasse (35).

5. Instrument nach einem der vorstehenden Ansprüche,
**dadurch gekennzeichnet, dass** die Gehäuseschalen (27-33) miteinander stoffschlüssig verbunden sind.

6. Instrument nach einem der vorstehenden Ansprüche,
**dadurch gekennzeichnet, dass** die Gehäuseschalen (27-33) jeweils eine einheitliche Wandstärke aufweisen.

7. Instrument nach einem der vorstehenden Ansprüche,
**dadurch gekennzeichnet, dass** das Innenteil (22) aus Metall besteht.

8. Instrument nach Anspruch 7, **dadurch gekennzeichnet, dass** das Instrument (21) mindestens eine Elektrode (16) aufweist.

9. Instrument nach Anspruch 8, **dadurch gekennzeichnet, dass** die Elektrode (16) von dem Innenteil (22) isoliert ist.

10. Instrument nach Anspruch 8, **dadurch gekennzeichnet, dass** die Elektrode (16) und das Innenteil (22) elektrisch miteinander verbunden und als ein Teil ausgebildet sind.

11. Instrument mit zwei Griffstücken (12, 13) jeweils nach einem der vorstehenden Ansprüche, **dadurch gekennzeichnet, dass** die Griffstücke (12, 13) aneinander schwenkbar gelagert sind.

12. Verfahren zur Herstellung eines elektrochirurgischen Instruments (11), bei dem mindestens ein Innenteil (22) und wenigstens zwei Gehäuseschalen (27-33) bereitgestellt und so zusammengefügt werden, dass die Gehäuseschalen (27-33) einen Innenraum (34) umgeben, in dem das Innenteil (22) angeordnet ist, wonach der Innenraum (34) mit einer Vergussmasse (35) ausgefüllt und die Vergussmasse (35) ausgehärtet wird, wobei zwischen den Gehäuseschalen (27-33) Fugen (36, 37) ausgebildet und die Gehäuseschalen (27-33) an den Stirnseiten (38, 39) profiliert sind, **dadurch gekennzeichnet, dass** die Fugen (36, 37) vollständig mit der Vergussmasse (35) gefüllt werden.

## Claims

1. An electrosurgical instrument (11), in particular thermally sterilizable instrument (11),
having at least one handle piece (12, 13), which has a housing (21) that is assembled from several housing shells (27-33) and that surrounds an inner part (22), wherein an interstice (34) is formed between the inner part (22) and the housing (21) and is filled with a casting compound (35), wherein joints (36, 37) are formed between the housing shells (27-33) and the housing shells (27-33) are profiled on the faces (38, 39), **characterized in that** the joints (36, 37) are completely filled with the casting compound (35).

2. The instrument according to claim 1, **characterized in that** the interior (34) is completely filled with the casting compound (35) free of air entrapments.

3. The instrument according to any of the preceding claims, **characterized in that** at least one electrical component (19, 47) is arranged in the interstice (34).

4. The instrument according to any of the preceding claims, **characterized in that** the housing shells (27-33) are made of a plastic which is stiffer than the casting compound (35).

5. The instrument according to any of the preceding claims, **characterized in that** the housing shells (27-33) are connected to one another by substance bond.

6. The instrument according to any of the preceding claims, **characterized in that** the housing shells (27-33) each have a uniform wall thickness.

7. The instrument according to any of the preceding claims, **characterized in that** the inner part (22) is made of metal.

8. The instrument according to claim 7, **characterized in that** the instrument (21) comprises at least one electrode (16).

9. The instrument according to claim 8, **characterized in that** the electrode (16) is insulated from the inner part (22).

10. The instrument according to claim 8, **characterized in that** the electrode (16) and the inner part (22) are electrically connected to each other and are formed as one part.

11. The instrument according to any of the preceding claims, comprising two handle pieces (12, 13) that are pivotally mounted on one another.

12. A method for manufacturing an electrosurgical instrument (11), in which at least one inner part (22) and at least two housing shells (27-33) are provided and are joined together such that the housing shells (27-33) surround an interior (34), in which the inner part (22) is arranged, after which the interior (34) is filled with a casting compound (35) and the casting compound (35) is cured, wherein joints (36, 37) are formed between the housing shells (27-33) and the housing shells (27-33) are profiled on the faces (38, 39), **characterized in that** the joints (36, 37) are completely filled with the casting compound (35).

## Revendications

1. Instrument électrochirurgical (11), notamment instrument (11) pouvant être stérilisé par voie thermique,
comprenant au moins un manche (12, 13) présentant un boîtier (21) qui se compose de plusieurs coques de boîtier (27-33) et qui entoure un élément interne (22), sachant qu'il est prévu, entre l'élément interne (22) et le boîtier (21), un espace (34) qui est rempli avec une masse de scellement (35), des joints (36, 37) étant formés entre les coques de boîtier (27-33), et les coques de boîtier (27-33) étant profilées sur les faces frontales (38, 39), **caractérisé en ce que** les joints (36, 37) sont entièrement remplis avec la masse de scellement (35).

2. Instrument selon la revendication 1, **caractérisé en ce que** l'espace interne (34) est rempli avec la masse de scellement (35) en étant totalement exempt d'inclusions d'air.

3. Instrument selon une des revendications précédentes, **caractérisé en ce qu'**au moins un composant électrique (19, 47) est disposé dans l'espace (34).

4. Instrument selon une des revendications précédentes, **caractérisé en ce que** les coques de boîtier (27-33) sont constituées d'une matière plastique qui est plus rigide que la masse de scellement (35).

5. Instrument selon une des revendications précédentes, **caractérisé en ce que** les coques de boîtier (27-33) sont reliées entre elles par matière.

6. Instrument selon une des revendications précédentes, **caractérisé en ce que** les coques de boîtier (27-33) présentent respectivement une épaisseur de paroi uniforme.

7. Instrument selon une des revendications précédentes, **caractérisé en ce que** l'élément interne (22) est constitué de métal.

8. Instrument selon la revendication 7, **caractérisé en ce que** l'instrument (21) présente au moins une électrode (16).

9. Instrument selon la revendication 8, **caractérisé en ce que** l'électrode (16) est isolée vis-à-vis de l'élément interne (22).

10. Instrument selon la revendication 8, **caractérisé en ce que** l'électrode (16) et l'élément interne (22) sont reliés électriquement l'un à l'autre et sont réalisés d'une seule pièce.

11. Instrument doté de deux manches (12, 13), respectivement selon une des revendications précédentes, **caractérisé en ce que** les manches (12, 13) sont montées l'une sur l'autre avec possibilité de pivotement.

12. Procédé de fabrication d'un instrument électrochirurgical (11), selon lequel au moins un élément interne (22) et au moins deux coques de boîtier (27-33) sont mis à disposition et assemblés de manière à ce que les coques de boîtier (27-33) entourent un espace interne (34) dans lequel est disposé l'élément interne (22), à la suite de quoi l'espace interne (34) est rempli avec une masse de scellement (35), et la masse de scellement (35) est durcie, sachant que des joints (36, 37) sont formés entre les coques de boîtier (27-33), et les coques de boîtier (27-33) sont profilées sur les faces frontales (38, 39), **caractérisé en ce que** les joints (36, 37) sont remplis complètement avec la masse de scellement (35).
